# EUROPEAN PATENT SPECIFICATION

(11) **EP 4 494 850 B1**
(45) Date of publication and mention of the grant of the patent: **01.04.2026**
(21) Application number: 24189728.9
(22) Date of filing: 19.07.2024
(51) Int. Cl.: B29C 65/08, A47G 9/02, B32B 5/02, B32B 37/00, B32B 37/06, B32B 37/14, B32B 37/20, B32B 37/04, B29C 65/74, B29C 65/00, A61F 7/02, B32B 5/06, B32B 5/26, B32B 27/12, B29L 31/48, A61F 7/00

(54) **LAYERED TEXTILE MATERIAL**
MEHRLAGIGES TEXTILMATERIAL
MATÉRIAU TEXTILE STRATIFIÉ

(30) Priority: 20.07.2023 GB 202311128
(43) Date of publication of application: 22.01.2025
(73) Proprietor: John Horsfall & Sons Limited, Huddersfield West Yorkshire HD2 2XB (GB)
(72) Inventor: FIELDS, Tony, HD2 2XB Huddersfield (GB); STAMP, Kevin, HD2 2XB Huddersfield (GB); CODD, Tony, HD2 2XB Huddersfield (GB)
(74) Representative: Appleyard Lees IP LLP

(56) References cited:
- CN-U- 204 132 990
- US-A1- 2018 263 385
- US-A1- 2019 343 202

## Description

### Field of the Invention

The present disclosure relates to a method of manufacture of a layered textile material, a resulting material thereof, and an apparatus for use in such a method. In particular the disclosure is concerned with the manufacture of a layered textile material for use in preventing hypothermia.

### Background

Hypothermia is a potentially very severe condition that can occur when a person's core body temperature falls below approximately 35 °C. Causes of hypothermia include exposure to cold weather or water, as well as interruptions to normal bodily thermoregulation arising from e.g., alcohol intoxication, low blood sugar, trauma, and so on.

A particular problem with treating hypothermia is how to do so in the field - i.e., when the patient is remote from advanced medical care (e.g., hospital).

One solution is to wrap a patient in a heated blanket, but this approach is problematic in locations without suitable power sources to heat such a blanket, and even if a portable power source is provided, they may be cumbersome, low on energy, prone to failure, etc. That is, the patient is warmed by a secondary heat source in an active warming process.

When onsite heating isn't available, a related approach involves wrapping the patient in a Mylar blanket. The Mylar blanket is reflective, and so stops the patient getting colder, but it also prevents them from warming up from external heat sources. As such, if the patient is already hypothermic, then they stay hypothermic, making transport to a medical centre all the more urgent.

Thus it is highly desirable to provide an improved blanket for treating hypothermia as an alternative to previous designs. Moreover, it is not straight forward to simply provide a blanket of suitable material which combats hypothermia, and so it is similarly desired to develop a reliable and robust manufacturing technique for such blankets.

In the related art, US2018/0263385 discusses a warming blanket having a metalized fabric exterior and method of manufacture thereof, US2019/0343202 discusses an insulating material and method of using that material for preventing perioperative hypothermia, and CN204132990U discusses a multilayer heat-sealing warming blanket.

### Summary

The present invention is defined according to the independent claims. Additional features will be appreciated from the dependent claims and the description herein. Any embodiments which are described but which do not fall within the scope of the claims are to be interpreted merely as examples useful for a better understanding of the invention.

The example embodiments have been provided with a view to addressing at least some of the difficulties that are encountered with production of current hypothermia blankets, whether those difficulties have been specifically mentioned above or will otherwise be appreciated from the discussion herein.

In one aspect of the invention there is provided a method of manufacture of a layered textile material for combatting hypothermia. The method comprises arranging a set of material layers comprising, in order, first to fourth layers. The first layer comprises an insulating fabric, such as a needle punch fabric, the second and fourth layers each comprises a thermoplastic, such as oriented Polypropylene, and the third layer comprises a thermally reflective metal, such as an aluminium foil. The method further comprises forming the layered textile material by ultrasonically welding the arranged set of material layers together, whereby the weld is applied to from about 4.7% to about 6.0% of the surface area of the arranged set of material layers, and further preferably from about 5.0% to about 5.5% of the surface area arranged set of material layers. In this way may a material be produced which has improved warming and heat retention properties compared to existing hypothermia blankets. By welding only a small area of the finished material, an amount of contact between different layers of the finished material may be reduced, which reduces the possibility of thermal leakage by heat conduction between the material layers.

In an example, ultrasonic welding of the set of material layers comprises contacting the set of material layers at points with diameter from about 1.5 mm diameter to about 1.7 mm diameter, for example 1.6 mm, which may result in a welded area of substantially 2 mm on the layered textile material. Again, small weld size equates to reduced thermal leakage, without being so small as to cause damage to the material during manufacture.

In an example, the ultrasonic welding comprises welding a diamond pattern into the layered textile material, with pockets between welds forming the diamond pattern optionally about 40 mm by 40 mm. This size of pocket allows the fibres within the inner lining (first layer) to better retain trapped air thereby aiding with heat retention.

In an example, the method further comprises arranging the first to fourth layers in between a fifth layer comprising a non-woven fabric and a sixth layer comprising woven polyester with the fifth layer proximate to the first layer (i.e., sandwiching the first to fourth layers). The additional layers provide additional benefits such as material strength and fireproofing.

In an example, the method further comprises preforming the second to fourth layers as a bonded laminate foil, prior to arranging with the first layer ready for ultrasonic welding. In the present example the second to fourth layers are similar enough that an alternative bonding process may be applied, making the ultrasonic welding method herein more convenient by reducing the number of individual layers to deal with.

In an example, the method may further comprise simultaneously cutting and sealing an edge of the formed layered textile material, to provide a neatly finished yet still welded at the edges material.

In a related aspect of the invention, there is provided an apparatus (i.e., an ultrasonic welder) for use in the above method which comprises an embossing roller and a horn. Suitably, the embossing roller comprises a plurality of touch points configured to, in combination with the horn, apply pressure to the set of material layers to weld a surface area from about 4.7% to about 6.0% of the arranged set of material layers.

In an example, the plurality of contact points are disposed on the embossing roller in a pattern in which up to eight contact points are in contact with the horn during a weld, each being from about 1.5 mm diameter to about 1.7 mm diameter. A size of the horn which engages the eight contact points may be substantially 200 mm in length. The number and size of the contact points relative to horn size has been optimised to yield a suitably strong weld at a suitable material throughput, without impacting material finish quality.

In an example, the apparatus may suitably comprise means to ultrasonically cut and seal an edge of the layered textile material.

As used herein, unless otherwise expressly specified, all numbers such as those expressing values, ranges, amounts or percentages may be read as if prefaced by the word "about", even if the term does not expressly appear. The term "about", or substantially, when used herein means +/- 5% of the stated value. Also, any numerical range recited herein is intended to include all sub-ranges subsumed therein, and the terms "from" and "to" a pair of values are intended to indicate such values are included in the range. Singular encompasses plural and vice versa. Additionally, although the present invention has been described in terms of "comprising", the processes, materials, etc detailed herein may also be described as "consisting essentially of" or "consisting of".

### Brief Description of the Drawings

For a better understanding of the present disclosure reference will now be made to the accompanying drawings, in which:
Fig. 1 shows an example layered textile material for combatting hypothermia comprising four material layers;
Fig. 2 shows an example layered textile material comprising six material layers;
Fig. 3 shows an example ultrasonic welder and resulting welded material;
Fig. 4 shows an example embossing roller of the ultrasonic welder in more detail;
Fig. 5 shows an example contact point pattern of an example embossing roller;
Fig. 6 shows an example horn of the ultrasonic welder;
Fig. 7 shows an example welding pattern resulting on the finished example material;
Fig. 8 shows an example cutting and sealing tool.

### Detailed Description

At least some of the following example embodiments provide an improved material for combatting hypothermia in a patient, and in particular an example method of manufacture of such a material. The example method reliably and robustly yields the example material which has the desirable thermal properties, as well as ancillary properties such as drape, desired from a hypothermia blanket. Other advantages and improvements will be discussed in more detail herein.

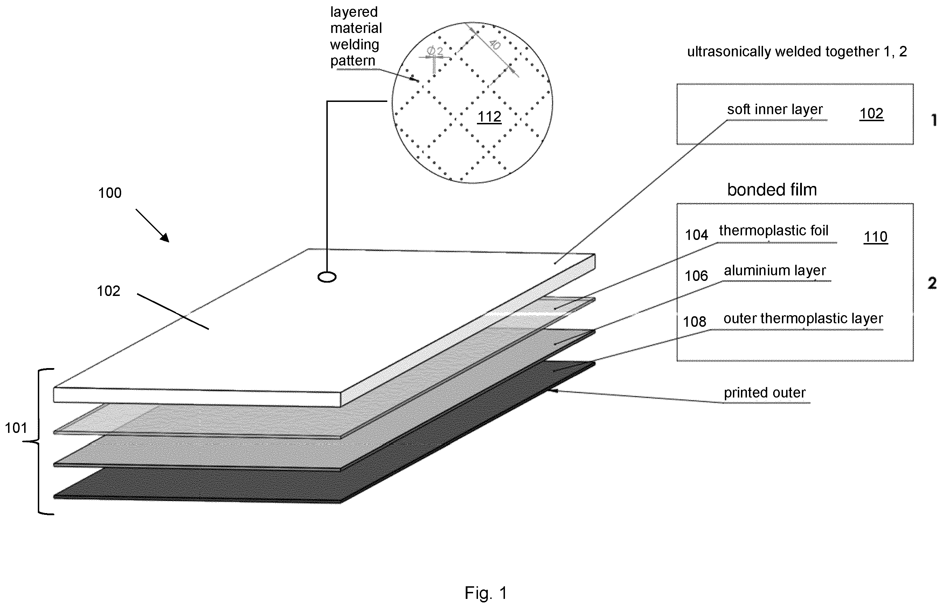

Figure 1 shows an example material 100 for use in a blanket (or other garment, etc) to combat hypothermia. The material 100 is formed from a set of four material layers 101, and may be suitably termed a layered textile material.

A first layer 102 in the set comprises an insulating fabric, such as a fibrous needle-punch polyester material, and is envisaged as the inner layer of the material when a blanket of the material is in use; that is, the first layer 102 may be the layer which abuts a patient's body when the material 100 is being worn. Needle punch materials offer optimum loft in order to trap, warm, and allow for movement of air.

Suitably, the first layer is provided for heat retention by retaining warm air within its inner fibres, and thereby allows for passive warming of the patient via convection. In other words, the first layer 102 provides some of the benefit of heated blankets, without requiring a secondary heat source (and so is passive in nature).

A second layer 104 in the set comprises a thermoplastic foil, which acts to provide the layered textile material 100 with suitable drape - that is, so that the material wraps around the patient, and doesn't leave large misshapen air pockets between the patient and blanket which would reduce warming efficiency. The second layer of thermoplastic also adds strength to the resulting material 100, as well as providing a moisture barrier. In one example the second layer 104 comprises oriented Polypropylene (OPP).

A third layer 106 comprises a thermally reflective metal which acts to reflect infra-red body heat; e.g., a metallised foil, such as an aluminium foil (or more generally a foil comprising aluminium). Suitably the third layer 106 maintains the warmth of the patient and prevents them getting colder, analogous to e.g., existing Mylar blankets.

A fourth layer 108 comprises another thermoplastic foil, which may be the same type of thermoplastic - e.g., OPP - to the second layer 104, or a different thermoplastic. Like the second layer 104, the fourth layer is provided to further improve the drape of the material 100. Suitably the fourth layer 108 may be an outer layer of the material 100; that is, the layer of the material 100 which is exposed to the elements when a blanket incorporating the material is wrapped around a patient. Suitably, another benefit of the thermoplastic layer 108 is to add an element of waterproofing and wind proofing to the material 100, as well as being non-conductive.

In an example, the second to fourth layers 104-108 - i.e., the foil layers - may be formed as a laminated foil. That is, the foil layers 104-108 may be considered a subset 110 of the set of material layers 101. In particular, the second to fourth layers 104-108 in (pre-formed) combination may be considered a metallised plastic foil, whereby the metal layer is sandwiched in between plastic layers.

Also shown in Fig. 1 is an example pattern 112 present on the layered textile, here a diamond pattern, which arises from the ultrasonic welding manufacturing process (discussed below). While diamond patterns are of course well known in the textile industry, the size of the pattern 112 in the present example is specifically chosen in order to provide air pockets (between pattern welds) that allows the fibres within the inner lining layer 102 to retain trapped air thereby aiding with heat retention within the inner layer 102. The diamond pattern 112 also provides a suitably uniformed means of bonding materials together that are used across a range of different products as well as giving a cosmetic design look. In the present example the diamond pattern provides an area between material bonds of substantially 40mm x 40mm.

It will of course be appreciated that other patterns are suitable dependant on the number of layers required, the blanket materials and desired final properties, and exact implementation of the manufacturing process.

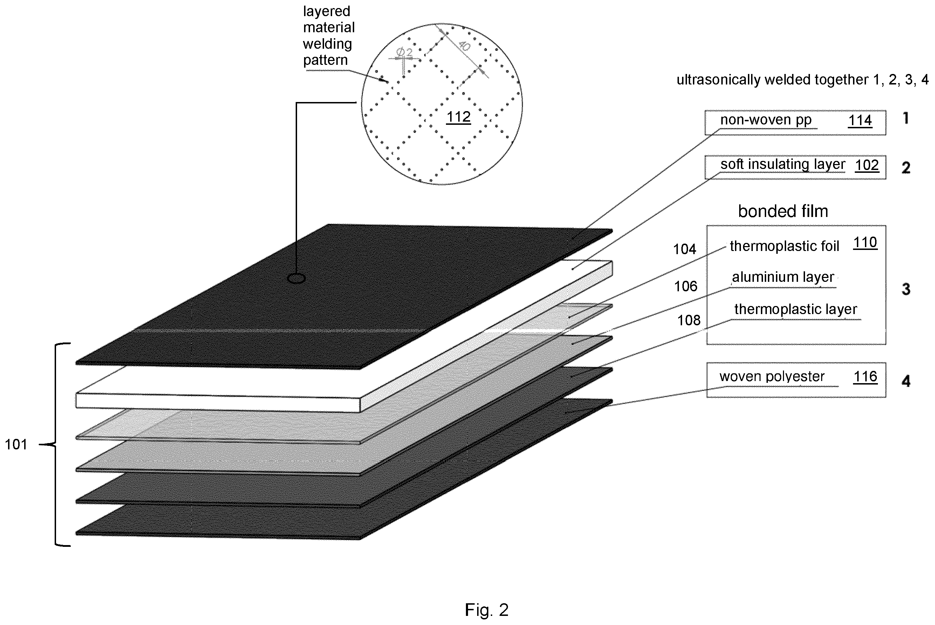

Figure 2 shows another example material 100, this time formed from a set of six material layers 101. In this example the same first to fourth layers are present as discussed above, sandwiched between a fifth layer 114 and a sixth layer 116. That is, here the example of Fig. 1 forms the core layers of the example of Fig. 2, with the fifth layer 114 proximate to the first layer 102, and the sixth layer 116 proximate to the subset of foil layers 110.

The fifth layer 114 may comprises a non-woven fabric, for example spun polypropylene. The non-woven fabric layer 114 provides additional insulation, in addition to providing the material 100 with a more robust inner layer (e.g., less prone to wear) and greater strength to the material 100. A further benefit of the non-woven fabric is that it provides a pleasant soft feel to the patient, and can be brightly coloured for easy identification of improper wrapping of a patient in the material (i.e., a visual check on whether there are gaps in how a patient has been wrapped).

The sixth layer 116 may comprise a woven polyester fabric, which adds durability, puncture resistance and mechanical strength to the material 100, in addition to further waterproofing and fire-resistance.

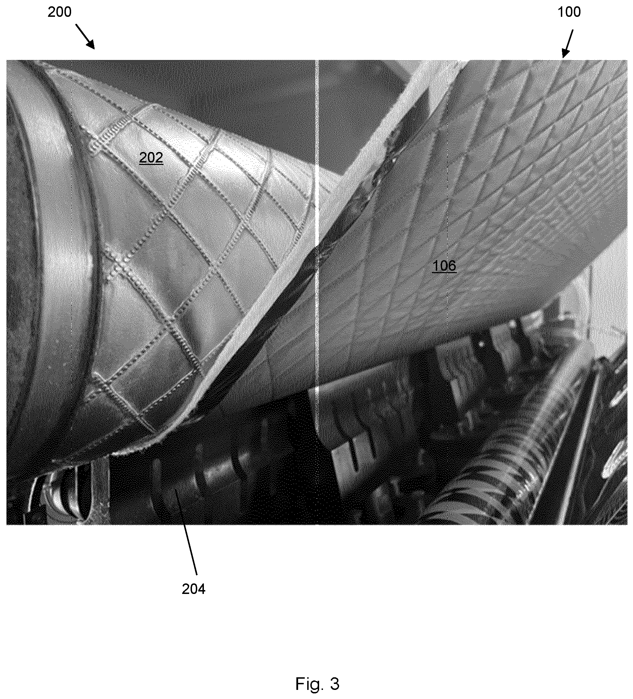

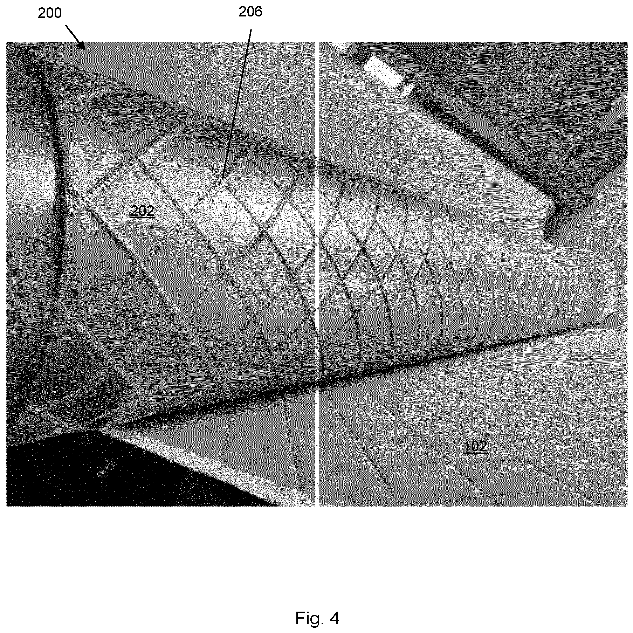

Figures 3 & 4 show an example ultrasonic welder 200 which is used for manufacturing the layered textile material 100. The ultrasonic welder comprises an embossing roller 202, at least one horn 204, and a feeder means (not shown), such as a conveyor, for feeding material between the embossing roller 202 and horn 204. While in practice the ultrasonic welder 200 comprises a plurality of horns, which may be substantially identical, for ease of explanation the following will discuss just a single horn 204.

Suitably, to manufacture the layered textile material 100, a set of material layers to form the material 100 are arranged, in order, (i.e., on the feeder means) and fed between the embossing roller 202 and horn 204 to be welded together. For the example material of Fig. 1 comprising the set of first to fourth layers, the order is first layer 102, second layer 104, third layer 106, fourth layer 108. For the example material of Fig. 2 comprising first to sixth layers, the order is fifth layer 114, first layer 102, second layer 104, third layer 106, fourth layer 108 and sixth layer 116. It will be appreciated that other material layers may be added to the set of layers for forming into the layered textile material 100, in between, before, or after any of the layers above, provided that at least the first to fourth layers are at least in the respective order.

In one example, each of the material layers 102-108 is arranged individually - i.e., layered one atop the other - prior to insertion to the ultrasonic welder 200. In a particularly convenient example, however, the subset of foil layers 110 - i.e., second layer 104, third layer 106, and fourth layer 108 - may be preformed as laminated foil. Being of similar materials, it may be desirable to pre-bond the foil layers using an alternative to ultrasonic welding, while using the ultrasonic welding only to weld the needle punch fabric (first) layer 102 (and optionally non-woven fabric fifth layer 114) to the pre-formed foil laminate 110 (and optionally sixth layer 116). Suitably, when the example material of Fig. 1 is to be produced, the method may comprise arranging two materials ready for welding (the first material layer 102, and the metallised plastic foil material 110). Likewise, when the example material of Fig. 2 is to be produced, the method may comprise arranging four materials ready for welding; separately the materials of the first 102, fifth 114, and sixth 116 layers along with the combined foil 110.

As will be familiar to those in the art, the horn 204 is configured to vibrate at high frequency in a direction towards the embossing roller 202 (for example, orthogonal to the curvature of the embossing roller), causing the set of material layers 102-108 therebetween to be welded together due to the pressure created on the material. In the present example the embossing roller 202 comprises a plurality of contact points 206 while the horn 204 is substantially flat and vibrated at approximately 20 kHz. Such a frequency has been found to be particularly advantageous in yielding a strongly welded finished material 100.

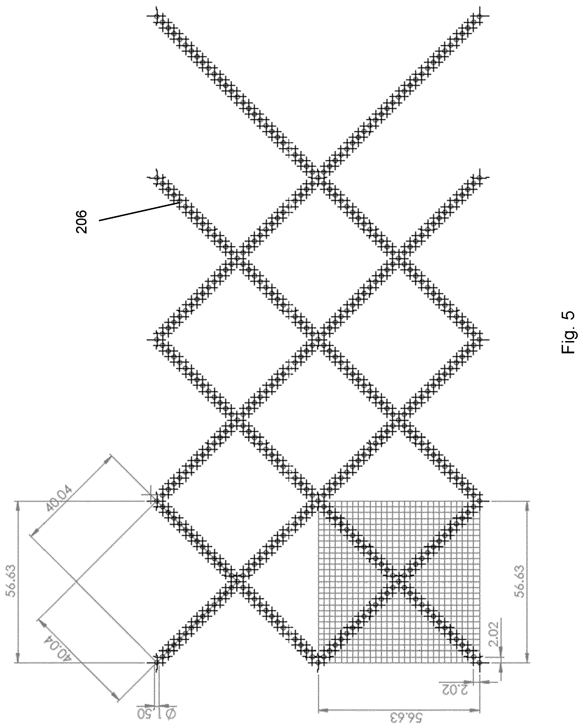

Figure 5 shows the pattern of contact points 206 in further detail. The contact points 206 are disposed on the embossing roller 202 such that they weld a surface area from about 4.7% to about 6.0% of the layered textile material 100. Suitably, during manufacture of the material 100, the set of material layers 102-108 are ultrasonically welded together by a welding area from about 4.7% to about 6.0% of the surface area of the layered textile material 100.

A 4.7% weld surface area may be suitable when first configuring the embossing roller, in order to account for a degree of wear on the contact points during the manufacturing process (which will flatten the contact points somewhat over time). Above the upper limit of about 6% surface area there is an unacceptable drop-off in welding throughput capacity as well as material bond strength, and so 6% weld area represents the manufacturing limit for the presently described material 100.

Importantly, by welding only a small area of the finished material 100, the amount of contact between different layers of the finished material 100 may be reduced, which reduces the possibility of thermal leakage by heat conduction between the material layers, thereby improving the efficiency of the finished material 100 when used in a suitable blanket for hypothermia treatment.

Preferably, the contact points 206 are disposed on the embossing roller 202 to weld from about 5% to about 5.5% of the material 100. Such a weld area yields an ideal trade off between machine speed, strong weld and reduced thermal leakage; by way of example, the presently described arrangement allows for a manufacturing speed of around 6 metres per minute on a welder 200 configured as discussed herein. Notably the bond strength starts to deteriorate beyond 5.5%, until the manufacturing limit of about 6% weld area.

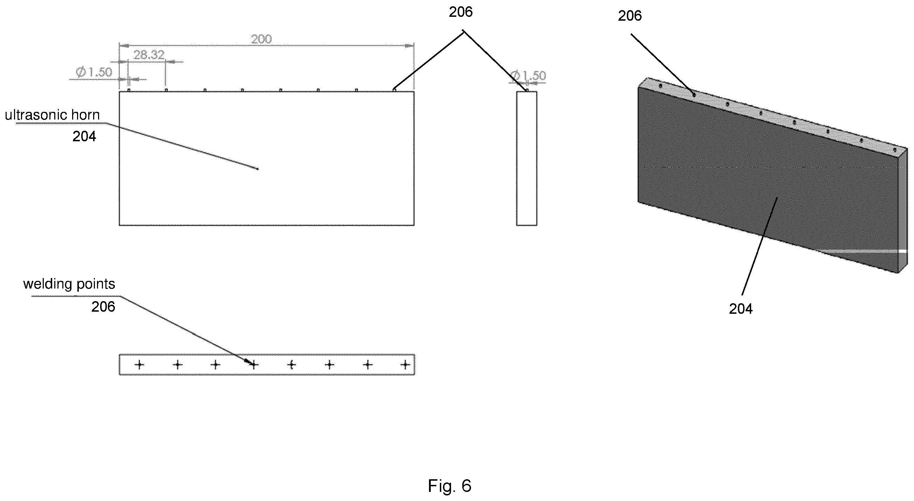

Figure 6 shows an example interaction between the contact points 206 and the horn 204. Here the horn 204 is substantially rectangular with major axis (longest side) length of approximately 200 mm. Each of the contact points 206 are disposed on the embossing roller with a suitable linear separation so that only 8 contact points 206 are engaged by the horn 204 at any one time. Here each contact point 206 is separated by approximately 28 mm from an adjacent contact point 206. That is, as the embossing rolling 202 turns and the horn 204 moves towards the roller 202, the material 100 is welded at 8 points disposed linearly in the major axis of the horn 204; in the next cycle, after the roller as turned by a certain degree, a different 8 points of material are welded by the horn 204.

Suitably, each of the embossing points may be sized from about 1.5 mm diameter to about 1.7 mm diameter in order to correspond to the substantially 200 mm length of horn 204. By way of examples, for 1.5 mm contact points 206, the 8 points have an area of 14.16 mm² across a 300 mm² area of horn 204 (1.5 mm width by 200 mm length) yielding a weld area of 4.72%; for a 1.6 mm diameter contact points the 8 points yield a weld area of 5.36%; and for a 1.7 mm diameter contact points 206 the 8 points yield a weld area of 6.05%. Suitably, for an 8 contact point arrangement on a 200 mm horn 204, a 1.6 mm diameter contact point is preferred to yield a weld area in the desired range of 5-5.5%.

It will be appreciated that other sizes of contact points 206, horn 204 dimension, etc, may be chosen to achieve the desired welding area. However, the smaller each individual contact point 206 (i.e., weld point), the higher number of contact points 206 will be required for a given line of material, which can cause problems for the manufacturing process. For example, a contact point dimension of 1.2 mm was tested and found to cause punctures through the material 100.

Considering the small size of the contact points 206 just described, the material 100 manufacturing process may be improved by arranging the first layer 102 or, if present the fifth layer 114 facing the embossing roller 202. In this way the initial contact of material 100 with the embossing roller 202 is absorbed by fibrous material, reducing the likelihood of damage to the foil layers 104-108 due to the small size of the contact points. As with providing a small weld area, ensuring small size of the contact points further recues the thermal bridging points for heat leakage between the material layers 102-108.

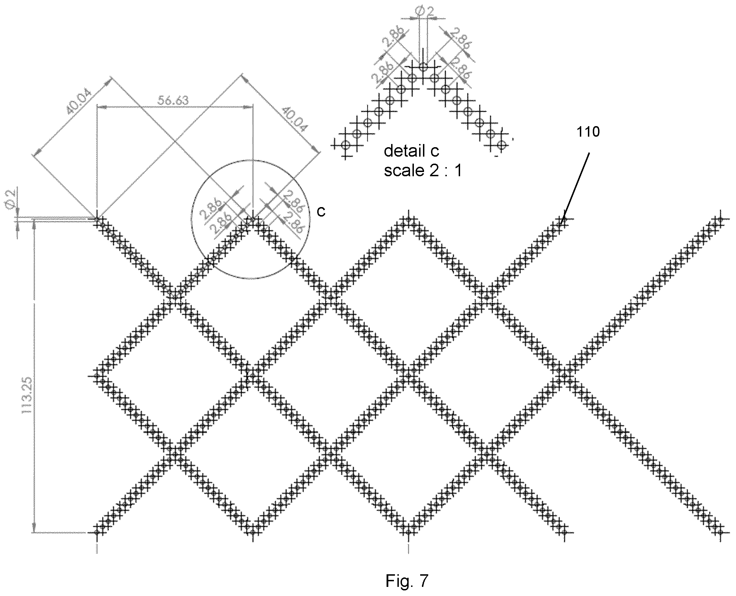

Figure 7 shows an example weld pattern 112 which results on the formed layered textile material 100 (as in also Figs. 1 & 2) from the embossing roller 202 just described.

Notably the finished material 100 has an average weld dot area which is greater than the dot size of 1.5-1.7 mm provided on the embossing roller 202 to produce the weld. This increase in size is down to a combination of roller usage and dots swaging over time, thermal heat transfer through the area around the weld, and also material stretch as the material runs across the ultrasonic horn 204 and partly around the embossing roller 202. Suitably, even with increase in size, the weld area of the finished material 100 is preferably from about 5% to about 5.5% surface area.

By way of example, for a preferred 1.6 mm contact point 206, the approximate welded area on a finished material 100 (using the diamond pattern) is about 2 mm. Taking a representative 113 mm by 113 mm square of material (as shown in Fig. 7 - area 12769 mm²), there are 224 weld dots within that area each having substantially 2 mm diameter. Thus the total welded area of the material is about 5.5% (increased from the initial 5.36% weld area).

In order to finish the layered textile material 100 into a suitable blanket/garment for treating hypothermia, it is desirable to cut the material 100 to a desired size. Preferably, the cut edge of the material 100 is cut and sealed in a single pass, so as contain any loose fibres and achieve a strong seal.

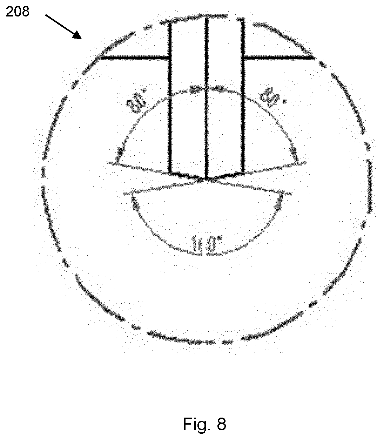

Figure 8 shows an example means 208 to ultrasonically cut and seal an edge of the layered textile material 100 (i.e., after it has been welded) in a single pass. In one example the cutting tool may be integrated into the ultrasonic welder 200 (i.e., controlled by the same control system). In another example, the cutting tool may be provided as a separate apparatus.

The tool angle may vary depending on material thickness and properties, and is dependent on a compromise between cleanly cutting through the layered materials and bonding the outer edge within one process. Suitably, the angle will need to be adjusted if a different foil or thickness is used. For the present examples, cutting tool 208 is provided with a tool angle of 160° ± 2°.

In summary, exemplary embodiments of an apparatus and method for manufacture of an improved material for combatting hypothermia have been described.

The example apparatus and method may be utilised industrially. An industrial application of the example embodiments will be clear from the discussion herein.

Although preferred embodiment(s) of the present invention have been shown and described, it will be appreciated by those skilled in the art that changes may be made without departing from the scope of the invention as defined in the claims.

Attention is directed to all papers and documents which are filed concurrently with or previous to this specification in connection with this application and which are open to public inspection with this specification, and the contents of all such papers and documents are incorporated herein by reference.

All of the features disclosed in this specification, and/or all of the steps of any method or process so disclosed, may be combined in any combination, except combinations where at least some of such features and/or steps are mutually exclusive.

Each feature disclosed in this specification may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s).

## Claims

1. A method of manufacture of a layered textile material (100) for combatting hypothermia, the method comprising:
arranging a set of material layers (101) comprising, in order, first to fourth layers (102 - 108), wherein the first layer (102) comprises an insulating fabric, the second (104) and fourth (108) layers each comprises a thermoplastic, and the third layer (106) comprises a thermally reflective metal; and
**characterised by** ultrasonic welding the arranged set of material layers (101) to form the layered textile material (100), wherein the ultrasonic welding of the set of material layers comprises welding a surface area from 4.7% to 6.0% of the arranged set of material layers.

2. The method of claim 1, wherein the ultrasonic welding comprising welding a surface area from 5.0% to 5.5% of the arranged set of material layers.

3. The method of any preceding claim, wherein the ultrasonic welding of the set of material layers (101) comprises contacting the set of material layers at points with diameter from 1.5 mm diameter to 1.7 mm diameter, for example 1.6 mm.

4. The method of any preceding claim, wherein the ultrasonic welding results in a welded area of substantially 2 mm on the layered textile material.

5. The method of any preceding claim, wherein the insulating fabric (102) comprises a fibrous needle punch material.

6. The method of any preceding claim, wherein the thermally reflective metal (106) comprises aluminium.

7. The method of any preceding claim, wherein the thermoplastic (104, 108) comprises oriented Polypropylene.

8. The method of any preceding claim, wherein the ultrasonic welding comprises welding a diamond pattern (112) into the layered textile material.

9. The method of claim 8, wherein pockets between welds forming the diamond pattern (112) are about 40 mm by 40 mm.

10. The method of any preceding claim, wherein arranging the set of layers (101) comprises arranging the first to fourth layers in between a fifth layer (114) comprising a non-woven fabric and a sixth layer (116) comprising woven polyester, the fifth layer being proximate to the first layer (102).

11. The method of any preceding claim, wherein the ultrasonic welding is performed at a frequency of substantially 20 kHz.

12. The method of any preceding claim further comprising preforming the second to fourth layers as a bonded laminate foil (110), prior to arranging with the first layer ready for ultrasonic welding.

13. The method of any preceding claim, further comprising simultaneously cutting and sealing an edge of the formed layered textile material.

## Patentansprüche

1. Verfahren zur Herstellung eines geschichteten Textilmaterials (100) zum Bekämpfen von Hypothermie, wobei das Verfahren umfasst:
Anordnen eines Satzes von Materialschichten (101), umfassend der Reihenfolge nach erste bis vierte Schichten (102 - 108), wobei die erste Schicht (102) ein isolierendes Gewebe umfasst, die zweite (104) und die vierte (108) Schicht jeweils einen Thermoplast umfassen und die dritte Schicht (106) ein wärmereflektierendes Metall umfasst; und
**gekennzeichnet durch** Ultraschallschweißen des angeordneten Satzes von Materialschichten (101), um das geschichtete Textilmaterial (100) zu bilden, wobei das Ultraschallschweißen des Satzes von Materialschichten Schweißen einer Oberfläche von 4,7 % bis 6,0 % des angeordneten Satzes von Materialschichten umfasst.

2. Verfahren nach Anspruch 1, wobei das Ultraschallschweißen Schweißen einer Oberfläche von 5,0 % bis 5,5 % des angeordneten Satzes von Materialschichten umfasst.

3. Verfahren nach einem der vorstehenden Ansprüche, wobei das Ultraschallschweißen des Satzes von Materialschichten (101) Inkontaktbringen des Satzes von Materialschichten an Punkten mit einem Durchmesser von 1,5 mm Durchmesser bis 1,7 mm Durchmesser, beispielsweise 1,6 mm, umfasst.

4. Verfahren nach einem der vorstehenden Ansprüche, wobei das Ultraschallschweißen einen geschweißten Bereich von im Wesentlichen 2 mm auf dem geschichteten Textilmaterial ergibt.

5. Verfahren nach einem der vorstehenden Ansprüche, wobei das isolierende Gewebe (102) ein faserförmiges Nadelstanzmaterial umfasst.

6. Verfahren nach einem der vorstehenden Ansprüche, wobei das wärmereflektierende Metall (106) Aluminium umfasst.

7. Verfahren nach einem der vorstehenden Ansprüche, wobei der Thermoplast (104, 108) orientiertes Polypropylen umfasst.

8. Verfahren nach einem der vorstehenden Ansprüche, wobei das Ultraschallschweißen Schweißen eines Diamantmusters (112) in das geschichtete Textilmaterial umfasst.

9. Verfahren nach Anspruch 8, wobei Taschen zwischen Schweißnähten, die das Diamantmuster (112) bilden, etwa 40 mm mal 40 mm betragen.

10. Verfahren nach einem der vorstehenden Ansprüche, wobei Anordnen des Satzes von Schichten (101) Anordnen der ersten bis vierten Schicht zwischen einer fünften Schicht (114) umfassend ein Vlies und einer sechsten Schicht (116) umfassend gewebten Polyester umfasst, wobei die fünfte Schicht benachbart zu der ersten Schicht (102) angeordnet wird.

11. Verfahren nach einem der vorstehenden Ansprüche, wobei das Ultraschallschweißen bei einer Frequenz von im Wesentlichen 20 kHz durchgeführt wird.

12. Verfahren nach einem der vorstehenden Ansprüche, ferner umfassend Vorformen der zweiten bis vierten Schicht als eine gebundene Laminatfolie (110) vor Anordnen mit der ersten Schicht, die zum Ultraschallschweißen bereit ist.

13. Verfahren nach einem der vorstehenden Ansprüche, ferner umfassend gleichzeitiges Schneiden und Versiegeln einer Kante des gebildeten geschichteten Textilmaterials.

## Revendications

1. Procédé de fabrication d'un matériau textile multicouche (100) pour combattre l'hypothermie, le procédé comprenant :
l'agencement d'un ensemble de couches de matériau (101) comprenant, dans l'ordre, des première à quatrième couches (102 - 108), dans lequel la première couche (102) comprend un tissu isolant, les deuxième (104) et quatrième (108) couches comprennent chacune un thermoplastique, et la troisième couche (106) comprend un métal thermo-réfléchissant ; et
**caractérisé par** le soudage par ultrasons de l'ensemble agencé de couches de matériau (101) pour former le matériau textile multicouche (100), dans lequel le soudage par ultrasons de l'ensemble de couches de matériau comprend le soudage d'une superficie de 4,7 % à 6,0 % de l'ensemble agencé de couches de matériau.

2. Procédé de la revendication 1, dans lequel le soudage par ultrasons comprenant le soudage d'une superficie de 5,0 % à 5,5 % de l'ensemble agencé de couches de matériau.

3. Procédé d'une quelconque revendication précédente, dans lequel le soudage par ultrasons de l'ensemble de couches de matériau (101) comprend la mise en contact de l'ensemble de couches de matériau à des points avec un diamètre de 1,5 mm de diamètre à 1,7 mm de diamètre, par exemple de 1,6 mm.

4. Procédé d'une quelconque revendication précédente, dans lequel le soudage par ultrasons a pour résultat une superficie soudée de sensiblement 2 mm sur le matériau textile multicouche.

5. Procédé d'une quelconque revendication précédente, dans lequel le tissu isolant (102) comprend un matériau fibreux aiguilleté.

6. Procédé d'une quelconque revendication précédente, dans lequel le métal thermo-réfléchissant (106) comprend de l'aluminium.

7. Procédé d'une quelconque revendication précédente, dans lequel le thermoplastique (104, 108) comprend du polypropylène orienté.

8. Procédé d'une quelconque revendication précédente, dans lequel le soudage par ultrasons comprend le soudage d'un motif en losange (112) dans le matériau textile multicouche.

9. Procédé de la revendication 8, dans lequel des poches entre des soudures formant le motif en losange (112) mesurent environ 40 mm par 40 mm.

10. Procédé d'une quelconque revendication précédente, dans lequel l'agencement de l'ensemble de couches (101) comprend l'agencement des première à quatrième couches entre une cinquième couche (114) comprenant un tissu non tissé et une sixième couche (116) comprenant du polyester tissé, la cinquième couche étant à proximité de la première couche (102).

11. Procédé d'une quelconque revendication précédente, dans lequel le soudage par ultrasons est réalisé à une fréquence de sensiblement 20 kHz.

12. Procédé d'une quelconque revendication précédente, comprenant en outre le préformage des deuxième à quatrième couches sous forme de feuille stratifiée liée (110), avant l'agencement avec la première couche, prêtes pour le soudage par ultrasons.

13. Procédé d'une quelconque revendication précédente, comprenant en outre la découpe et le scellage simultanés d'un bord du matériau textile multicouche formé.
